# EUROPEAN PATENT APPLICATION

(11) **EP 1 063 289 A1**
(43) Date of publication of application: **27.12.2000**
(21) Application number: 99112279.7
(22) Date of filing: 25.06.1999
(51) Int. Cl.: C12N 5/08, A61F 2/02, A61M 1/36

(54) **Liver cell clones for artifical liver and extracorporeal liver assist device**

(71) Applicant: JMS Co., Ltd., Hiroshima-shi (JP)
(72) Inventor: Qiang,Shi Dr., Tianjin, 300071 (CN); Klinkmann, Horst, 18059 Rostock (DE); Kimura, Hajime, Tokyo-To (JP); Kazuo, Izumi, Hiroshima-Shi (JP)
(74) Representative: Winkler, Andreas, Dr.

(57) **Abstract**

Disclosed is a blood perfusion device or apparatus that is used for bioartificial liver support. Human hepatocyte lines established from normal regenerating liver tissue and modulated in toxin-challenging conditions are provided. These functional hepatocytes exhibit extraordinarily enhanced detoxification functions, which are characterised by the elevated glutathione content and glutathione S-transferase activity. A bioreactor is constructed with the functional hepatocytes for bioartificial liver support system, which includes perfusion inlets and perfusion outlets, a containment vessel, a centrifugal pump and macroporous microcarriers where the functional hepatocytes are grown.

## Description

### 3. Detailed Explanation of the Invention

### (Invention applicable industrial field)

The present invention is a specific extracorporeal liver assist device (sELAD) which is manufactured based on a novel human hepatocyte cell line (DYD) and macroporous microcarrier culture. The human hepatocyte cell line is established from human regenerating liver tissue and modified by enhancing specific detoxification functions in vitro, which is characterised by a higher GSH content and higher GST activity than its parental cell, besides expressing many of the characteristics of normal human hepatocytes.

This invention also relates to a method for blood detoxification or purification with emphasis not only on the toxins being recognised as potential toxins such as ammonia, phenols or middle molecules, but also on the toxins from hepatic necrotic tissue such as electrophilic metabolites and oxidants.

### (Prior art)

The mentioned functional hepatocytes are especially featured in detoxifying toxins from necrotic liver tissue, which is considered to be one of the most important factors in the development of hepatic encephalopathy in fulminate hepatic failure. The mentioned hepatocytes are grown on the macroporous microcarriers resulting in a 2.5 - 7.5 x 10¹⁰ accommodation per bioreactor module. The improvement both in quality and quantity of inoculated cells in the bioreactor makes the sELAD an attractive therapeutic remedy to treat the patient with fulminate hepatic failure.

Over the past several years, the focus of investigations on extracorporeal liver support system for treatment of fulminant liver failure has been shifting from experimental and animal trial to clinical evaluation and application. There are at least four groups trying their bioartificial liver devices under the supervision of FDA in United States (Rozga J et al, J Ann Surg 219:538-46 1994, Sussman NL et al, Amer J Kid Dis 18:371-84 1996, Sussman NL, Clin Invest Med 19:393-9 1996, Dixit V Scan J Gastroenterol Suppl 220:101-14 1996). Several groups from Europe have started their preclinical trials in Gemany and Netherlands, Great Britain, besides some early reports from Russia ( Gerlach J, Transplant Proc 29:852 1997, Hughes R.D et al, Semin Liver Dis 16:435-444 1996). Researches on bioartificial liver support device are also quite active in Japan.

The immediate objective of temporary liver support is to maintain a patient with acute or fulminant hepatic failure until its own liver regenerates. From the clinical data available, it appears that liver support systems are mainly used to bridge the patient to orthotopic transplantation, to improve cerebral circulation and hemo-dynamic parameters. Rozga and his group reported that among 19 patients treated with the use of bioartificial liver (BAL) device for 7 hours period on one to five occasions, 13 patients survived after liver transplantation at a mean of 39 hours after initiating BAL support. Patients who were not candidates for transplantation died (Kamlot A and Rozga J, Biotech & Bioeng 50:382-91 1996). The clinical assessment was carried out in 11 patients with acute liver failure who were given treatment with extracorporeal liver assist device (ELAD) for up to 114 hours. Nine often patients showed improved galactose elimination capacity, 1 recovering without transplantation, 4 undergoing transplantation, and others died (Sussman NL et al, Amer J Kid Dis 18:371-84 1996). Only in occasional cases did it show that patients recovered completely by the application of bioartificial liver support system. There were also some sporadic clinical reports dealing with the application of hepatocyte bioartificial liver treatment, the results were nearly the same to those mentioned above (Sheil AGR et al, Aust N Z J Surg 66:547-52 1996; Watanabe FD et al, Ann Surg 225:484-91 1997). Although hepatocyte-based hybrid extracorporeal liver support devices have demonstrated significant metabolic functions in a variety of experimental and animal tests, the results could not be confirmed completely in human subjects. Especially, biochemical measurements and efficiency demonstrated in patients with hepatic failure so far with two main well-known system are not so striking as did in animal tests. The clinical beneficial effects from these systems are prolonged survival rate, corrected biochemical abnormalities and increased toxins clearance. A well controlled clinical trial carried out recently in U.K to assess the value of ELAD (Sussmann) and BAL (Rozga) device, showed that with the two systems enhancement of certain metabolic functions in the patient have been observed, but the magnitude of the effects observed was not great (Hughes R.D et al, Semin Liver Dis 16:435-444 1996). This suggests that the functional capacity of the current devices be limited in clinical use at present, particularly considering the number of functions needing replacement in fulminant patient.

In the development of an extracorporeal liver support device, we are currently facing both theoretical and technical challenges to produce practically set-up for routine clinical application. These obstacles include insufficiency of basic pathophysiology which characterizes our knowledge of this area; incomplete understanding of liver biology and proliferation regulation of hepatocyte; especially mechanisms of hepatic coma or encephalopathy development in fulminant hepatic failure patient. Technically, either the quantity of the hepatocytes or their performance in the constructed bioreactor is not sufficient to produce clinically sufficient effects to correct the falling liver functions in fulminant hepatic failure patient. At present, primary cultures from animal mainly pig or immortalized cells from human hepatocytes constitute dominantly the biological components of most biological devices. These two types of cells are usually at opposite ends of a biologic continuum, because differentiation and proliferation are incompatible in most cell types according to the basic principles of biology. That is why it is so difficult to obtain large number of hepatocytes with desired multiple functionalities. The device containing about 50 g primary cultures from pig liver in the BAL system seems lower than that physiologically minimal mass (ranging from 200 - 400 g). In a higher number device with 400 g of C3A immortalized cells in the ELAD, major synthetic and metabolic functions were not demonstrated in clinical application (Hughes RD et al, Liver Transpl Surg 1:200-6 1995). Even high accommodation of primary culture of hepatocytes in a complex and well- designed configuration, good functions can only be found for a limited period of about 7 hours (Galletti PM & Jauregui HO, in Handbook of Bioengineering ppl952-66 1995). This makes continuous support of the patient with fulminant hepatic failure impractical by using fresh pig livers. It seems that no available method can prevent hepatocyte from deteriorating in vitro. Although great efforts have been made to improve the performance of hepatocytes including primary culture from xenograft and established cell line, no report claimed that they had maintained the differentiated hepatocytic functions significantly in bioreactors for bioartificial liver support system. Loss of hepatic functions in culture condition appears to be associated with cellular respones involving cellular interactions and cellular apotosis in vitro(Collins BH et al, Transplantation 58:1162-71 1994). Cells isolated from body are maintained in a non-physiologic environment and are cut off from numerous regulatory mechanisms which control hepatocyte function in vivo. It is reasonable to say, that hepatocyte bioreactors for bioartificial liver support have reached their maximal performance at present unless there is a radical theoretical and technological breakthrough to harvest multiple functional hepatocytes.

Another important issue is that bioartificial liver systems fail to show a significant advantage over artificial liver system. Some artificial liver system based on modern membrane technology and adsorbents have already been the subject of clinical assessment (Ash SR et al, Intl J Artif Org 15:151-61 1992). Highly adsorptive dialysis membrane coated with albumin is reported to facilitate the removal of potential toxins (Stange J et al, Artif Org 17:809-13 1993 ). BAL system still uses traditional charcoal adsorbents in the plasma circuit before the hepatocyte bioreactor (Rozga J & Demetriou AA, ASAIO J 41:831-37 1995). It is difficult to estimate what percentage of charcoal column functions in the system.

It is because the vast and pressing demand for artificial liver support that made bioengineers and clinicians invent various designs of device in spite of insufficient knowledge in understanding path-physiology of hepatic failure and simplify the treatment remedy for the patients. Both total artificial and bioartificial liver support system have been aiming at removal of a dozen of potential toxins recognized as factors causing encephalopathy in hepatic failure patient by adsorbents, dialysis and viable hepatocytes, however, the outcome of patient is not always proportional to their levels in patients' circulation. It remains uncertain whether hepatic coma is due to one or synergistic effects of these recognized metabolic abnormalities.

In addition to the toxic substances entering into the systemic circulation because of hepatic failure to clear the substances from portal blood by hepatocytes, toxins accumulated in patient's circulation also come from the hepatic necrotic tissue. And less is known about the nature and role of the necrotic substances in the development of encephalopathy. There are some indirect evidence showing that toxins from necrotic tissues have a critical influence on the clinical manifestations of acute hepatic failure at some stage. When necrotic tissue is removed from patient body, i,e, hepatectomy is carried out before transplantation, improvement in systemic hemodynamic parameters (systemic vascular resistance and oxygen utilization) and reduction in intracranial pressure (ICP) can be achieved (Ringe B et al, Transplant Proc 20:552-7 1988; Harrison PM et al, Gut 32:A837 1991). Moreover, if a patient treated with an extracorporeal liver support for a period of time and then followed orthotopic liver transplantation, expectation to the support is more satisfactory than those without transplantation because the necrotic liver is not removed. The mechanisms involved in liver injury are complex and interactive. Liver cells are damaged either by chemical poisoning or by immunologic cytotoxicity. Toxic metabolites from necrotic tissues may alter plasma membrane, mitochandria, intracellular ion homeostasis, or degratative enzyme activity in chemically induced damages, while immune mechanisms that involve cell interaction are mediated by cytokines, nitric acid and complement components. Both of them result in cellular integrity damage. It has been recognized that all these reactions are characterized by the production of active oxidants and reactive substances, subsequently promotes apoptosis of hepatocytes, which exacerbates load of active oxidants because liver is the largest organ to deal with or eliminate them. Oxidative stress results in mitochandria dysfunction, membrane injury and denaturation of DNA and other cell components (Sewell RB et al, Clin Sci 63:237 1982). Excessive production of oxygen radicals leads to altered enzymatic activities, decreased DNA repair, impaired utilization of oxygen, glutathione depletion and lipid peroxidation. The circulating toxins can cause pathological changes such as inflammatory reactions in all part of body, but cerebral cells are the most sensitive part to these toxins, thus, result in cerebral edema featured by increased ICP. Should it not treat properly, the clinical course is often complicated by multiple organ failure(MOF), with onset of severe clotting abnormalities, renal failure, pulmonary edema or cardiovascular collapse. Therefore, it is inferred that encephalopathy is an early symptom of multiple organ failure caused by toxins released from necrotic liver tissue. Although there is a few reports to indicate the involvement of active oxygen species toxins from necrostic tissue in the development of hepatic coma, it is inevitable that eradication of necrostic toxins would be an alternative to bioartificial liver support, and will play an increasing part in future design of bioreactor for liver support system.

We must admit the fact that global replacement by either primary culture or established cell line from human source is difficult to obtain a clinically significant effective device, at least the time being, as we know little how complex the multiple cellular interactions are and how to culture these differentiated cells. Numerous experiments have been done to prove that it is also impractical to mimic the hepatic functions with our current technologies. However, the past experiences with totally artificial systems indicated that detoxification by removal of toxins in the patient circulation had to a variable extent beneficial effects to patients' survival. This suggests that an extracorporeal artificial liver support system must not necessarily require all the multifunctional replacement by hepatocytes in the bioreactor, and that supplement of some specific functional activities that happen to be missing or deficient in the patients might be an alternative to obtain therapeutic effects from an artificial liver support.

However, which of the many diverse functions need to be replaced for fulminant hepatic failure is perhaps the most fundamental and important question to be considered. We do not know exactly which of the main categories, synthetic, bio-transformatory or excretory functions is critical for the recovery of hepatic coma. It is quite known that acute liver failure is usually associated with hepatic encephalopathy, and that it results in a high mortality. The main purpose of utilizing artificial liver support system is to prevent fulminant hepatic failure patient from development of encephalopathy, or treat it. The reversible and global nature of hepatic encephalopathy strongly indicate that a metabolic abnormality rather than direct toxic injury is involved in the pathogenesis of encephalopathy. And there is also a lot of evidence to show many toxins accumulating in the patient circulation upon the hepatic failure occurrence are responsible for the initiation of encephalopathy. Therefore, detoxification or removal of toxins in patients with fulminant hepatic failure is considered to be the primary task for the extracorporeal device. But, the facts that removal of toxins from patients by adsorbents or dialysis do not significantly improve survival in patient with severe encephalopathy strongly suggest that blood purification would be more specific and selective. And it would be less effective without the involvement of enzymatic transformations in living hepatocytes.

Detoxification by hepatocytes means that metabolites, toxins and the other harmful substances are transformed enzymatically into easily soluble compounds by cytochrome P-450 oxidation, and various conjugations. It represents one of the most important differentiated functions of hepatocyte. It is easily lost in cell culture and is reduced in transformed cell lines. Drug metabolizing cytochrome P-450 enzyme activity is a key activity and possible one of the most difficult to replace. The ability to perform conjugations is important in the detoxification of bilirubin, bile acids, phenols and a wide range of metabolites. However, some researches have indicated that these activities can be inducible to some extent when cells are encountered in different culture conditions.

### (Subjects which the invention intended to solve)

Since it is difficult to obtain extracorporeal liver assist device with all of the biochemical potential of hepatocytes in vivo, we could try to maintain some detoxification functions in the ELAD, or to increase the specific functional activities that are important and beneficial for the recovery of patient.

The present invention is to describe a method and a device or apparatus to enhance detoxification activity in immortalized human hepatocytes by a cellular approach, so that the specific functional cells can be easily obtained in a large mass equivalent to their counterpart in vivo.

### (Means to Solve the Subjects)

Considering hepatic detoxification function, the glutathione and glutathione S-transferase system (GSH/GST) plays an important part in removing toxic substances accumulating in the circulation of liver failure patients. GSH is the most prevalent low molecular weight peptide which has multiple physiological and metabolic functions. GSH participates in reactions that destroy hydrogen peroxide, organic peroxides, free radicals and foreign compounds or drugs. GSH also participates by a number of chemical mechanisms in the metabolism of various endogenous compounds. GSH/GST pathway represents part of an adaptive response mechanism to chemical stress caused by electrophiles, which are activated forms of most metabolites. Induction of GSH/GST pathway appears to detoxify some of the toxic carbonyl-, peroxide-, epoxide-containing metabolites produced within the cell by oxidative stress (Meister A. in The liver: Biology and Pathology, 1988 by Raven Press, Ltd.,New York). Since GSH functions in protecting cells from reactive oxygen intermediates, free radicals and toxic compounds, the increase in cellular GSH level and GST activity may be beneficial under certain conditions. Some clinical studies have shown that liver damage can be prevented and minimized by the application of glutathione precursor (Prescott LF et al, Lancet 2:109-13 1976; Rumack BH et al, Arch Intern Med 141:380-5 1981; Smilkstein MJ et al, N Engl J Med 319:1557-62 1988). Cellular GSH level may be partially increased by supplying substrates for enzymes in the GSH synthesis. However, the constitutive up-regulation of GSH synthesis and GST activity can be achieved by modulating the cells under a progressively toxin-challenging culture (Hamilton TC, in Glutathione S-Transferase Structure, Function and Clinical Implications, pp 173-85 1996). So does the induction of cellular defense system to the oxidants.

Modulation of intracellular GSH content and GST activity is going to be carried out in an immortalized hepatic cell line DYD, which is a human hepatocyte clone established from normal liver tissue. It is a highly differentiated cell line capable of growing at a high density. By endowing them with specific detoxification functions, the cells have ability to transform toxins more specifically, rapidly and efficiently, thus elevating the efficiency of bioreactor for bioartificial liver support system.

Quantity of hepatocytes inoculated in bioreactor is another important factor which influence the efficiency of current bioreactors. Although no one can figures out the exact number of hepatocytes in a bioreactor, the general acceptance of the cell mass is 100 - 300 g as extracorporeal support. The amount of cells in a single bioreactor is intended to reach 2.5 - 7.5 billion magnitude in this invention. In order to accommodate such a huge number of cells, a special bioreactor configuration is going to be constructed. The cells are allowed to grow to confluence on macroporous microcarriers, then remove cell-attached microcarriers into a bioreactor, where a continuous supply of nutrition and oxygen is guaranteed to maintain the steady functioning of the hepatocytes.

In order to modulate the detoxification function, highly differentiated human hepatocytes are prerequisite to an ideal liver support system. An immortalized cell line with highly differentiated functions is an attractive approach. Besides the unlimited cell division capacity, immortalized human hepatocyte obviate concerns about species-specific metabolic differences, and the infusion of human proteins is less likely to cause immune-mediated reactions, especially after prolonged or repeated use. Establishment of such kind of cell line is available with the advanced technology in cell and tissue culture. However, in order to obtain a clonal expansion of hepatocyte from normal liver tissue, special procedures in developing cell line are necessary, such as the use of new type matrix, growth factors or conditioned medium and induction of clonal expansion.

Enhancement of differentiated hepatocyte functions, specific to detoxification of hepatocyte, can be brought about by the induction and regulation of glutathione S-transferase, which is considered to represent a major group of detoxification enzymes. All eukaryotic species possess multiple cytosolic and membrane-bound GST isoenzymes, each of which displays distinct catalytic as well as non-catalytic binding properties(Hayes JD et al, Crit Rev Biochem Mol Biol 30:445-600 1995). Through the concerted actions of several isoenzymes, the GST supergene family provides several tiers of defense against toxic chemicals. Evidences suggests that the level of expression of GST is a crucial factor in determining the sensitivity of cells to a broad spectrum of toxic chemicals.

GST activity is increased in many organisms following exposure to foreign compounds. Induction of GST has been most thoroughly studied in rodents and at least 100 different chemicals have served as inducing agents of rat and mouse GST. The spectrum of xenobiotics that use as inducing agents, suggest that GST induction is part of an adaptive response mechanism to chemical stress that is widely distributed in nature. From studies of rodents, the adaptive response to chemical stress is clearly pleiotropic in character and involves the induction of many drug-metabolizing enzymes. Collectively, these detoxification enzymes provide protection against a diverse spectrum of harmful compounds. Besides these effects, evidences also suggest that GSTs are involved in protection against oxidative stresses (Lenehan PF et al, Cancer Chemother Pharmacol 35:377-86 1995).

It is known that some enhancers can regulate GST expression in the animal cells. Some enhancers interact with GST gene elements that respond to xenobiotics. They transcriptionally activate GST genes through different mechanisms according to their elemental structure (Hayes JD et al, Critical Reviews in Biochemistry and Molecular Biology 30: 445-600, 1995). The constitutive expression of GST up-regulation can be achieved by the effective modulations.

The immediate objective of an extracorporeal liver support system is to maintain a patient with fulminant hepatic failure until his own liver regenerates or to bridge the patient to orthotropic transplantation. Most liver support systems used rely on blood detoxification because it is considered the essential requirement for an extracorporeal liver support system. The metabolism of toxic substances by living cells in a hepatocyte bioreactor will reduce toxicity, thus produces the beneficial effects for the recovery of patients. This invention describes a novel detoxification system based on a special functional hepatocyte cell line, called specific Extracorporeal Liver Assist Device. The term "specific Extracorporeal Liver Assist Device" (sELAD) represents an unique extracorporeal liver support system. The said "specific" means that an extracorporeal liver support system provides extraordinarily some well-defined functional activities which are decreased but extremely important in the recovery of hepatic coma in fulminant hepatic failure patients. Since it is difficult to obtain an extracorporeal liver support system with all of the biochemical potential of hepatocytes in vivo, this invention introduces the system that possesses some of the main hepatic functions, preferentially detoxification as the feature of this system.

The term "specific Extracorporeal Liver Assist Device" as used herein also refers to an extracorporeal liver support system that possesses enhanced or elevated detoxification function, as hepatocytes inoculated in this system have several times higher content of detoxification enzymes and detoxificants than freshly isolated or transformed hepatocytes. The bioartificial liver support system using the principles of the present invention provides an effective alternative to treat patients with fulminant hepatic failure. The device and method of the present invention can be used not only in bridging the patient to orthotopic transplantation but also in preventing the patient from development of encephalopathy.

The pathway to enhance the specific detoxification functions in hepatocyte described in this invention is to modulate glutathione (GSH) and glutathione S-transferase (GST) levels in an immortalised human hepatocyte line (DYD), so that specific functional hepatocytes can be easily obtained in a sufficient mass equivalent to the counterpart in vivo.

### (Function)

Considering hepatic detoxification function, glutathione and glutathione S-transferase system (GSH/GST) plays an important part in removing toxic substances accumulating in the circulation of liver failure patients. Eukaryotic cells have evolved several mechanisms to protect cellular constituents from highly reactive molecules entering or being biotransformed in the cells. The greater affinity of electrophiles for thiol groups than for hydroxyl or amine groups provides a chemi-physical basis that high concentrations of thiol could be protective rather than the others. GSH is the most prevalent low molecular weight peptide which has multiple physiological and metabolic functions. GSH participates in reactions that destroy hydrogen peroxide, organic peroxides, free radicals and foreign compounds or drugs. The formation of a thioether bond between electrophiles and GSH almost yields a conjugate that is less reactive than the parental compound, therefore, the actions of GSH generally result in detoxification. GSH conjugation with harmful electrophilic moieties from the cell will increases the solubility of hydrophobic xenobiotics, which can be transported easily out of cells by ATP-dependent glutathione S-conjugate efflux pump (GSH-Px).

The conjugation reaction between GSH and xenobiotics represents the first step in the synthesis of mercapturic acids, an important group of excretion products. Following conjugation with GSH, the subsequent steps in mercapturic acid biosynthesis require the serial actions of glutamyl transpeptide, cysteinyl glycinase, and N-acetyl transferae, to the final form in urine. Since GSH functions in protecting cells from reactive oxygen intermediates, free radicals and toxic compounds, increase in cellular GSH level and GST activity may be beneficial under certain clinical conditions, such as hepatic coma in which a lot of toxins from necrotic tissue flushes into blood stream.

### (Examples)

The constitutive up-regulation of GSH synthesis and GST activity can be achieved by modulating the cells under a toxin challenging culture condition. The chemicals that induce GST and GSH are extremely diverse. These include carcinogens, cytotoxins, chemo-therapeutic drugs and heavy metals and metal-containing drugs. GST induction can also be performed by reactive oxygen species. Examples of these chemicals are *N*-acetoxy-2-amino-1-methyl-6-phenylimidazo[4,5-*b*]pyridine, aflatoxin B₁-8,9-epoxide, benzo[a]pyrene-4,5-oxide, benz[a]anthracene-5,6-oxide, benz[a]anthracene-8,9-diol-10,11-oxide, butadiene monoepoxide, +*anti* chrysene-1,2-diol-3,4-oxide, 5-hydroxymethylchrysene sulfate, 7,12-dihydroxymethylbenzo[a]anthracene sulfate, 7-hydroxymethyl-12-methylbenz[a]anthracene sulfate, 1-methyl-2-nitro-1-nitrosoguanidine, 1-nitropyrene-4,5-oxide, 1-nitropyrene-9,10-oxide, 4-nitroquinoline 1-oxide, benzo[a]pyrene, dimethylaminoazobenzene, 7,12-dimethylbenz[a]anthracene, 5,9-dimethyl-7H-dibenzo[*c,g*]carbazole, 3-methylcholanthrene, acetochlor, acifluorfen, alachlor, aldrin, atrazine, azinphosmethyl, 1,4-benzoquinone, chlorimuron ethyl, cumen hydroperoxide, DDT, *N,N*-diallyl-2-chloroacetamide, diazinon, dichlobenil, dichlofluanid, 2,4-dichlorophenoxyacetic acid, *S*-ethyl *N,N*-dipropylthiocarbamate sulfoxide, ethylene oxide, ethylparathion, fenoxaprop-ethyl, fluorodifen, lindane, malathion, methyl bromide, methyl chloride, methyl parathion, metolachlor, *trans*, *trans*-muconaldehyde, naphthaline 1,2-oxide, parathion, propachlor, propetamphos, styrene oxide, tetrachlorvinphos, *trans*-stilbene oxide, tridiphane, vinyl chloride, ampicillin, fosfomycin, penicillin, 1,3-*bis*(2-chloroethyl)-1-nitrosourea, chlorambucil, cyclophosphamide, ethacrynic acid, mechlorethamine, melphalan, mitozantrone, nitrogen mustard, thiotepa, acrolein, adenine propenal, cholesterol -ox i de, cytosine propenal, dilinoleoylphosphatidylcholine hydroperoxide, dilinoleoylphosphatidylethanolamine hydroperoxide, dilinoleoylphosphatidylglycerol hydroperoxide, epoxyeicosatrienoic acid, 4-hydroxynonenal, linoleic acid hydroperoxide, methyl linoleate ozonide, thymine propenal, uracil propenal, chlorotrifluoroethane, 1,4-dibromo-2,3-epoxybutane, dibromomethane, 1,3-dichloroacetone, dichloroacetylene, dichloromethane, 1,2,3,4-diepoxybutane, 1,2-epoxy-4-bromobutane, ethylenedibromide, hexachlorobutadiene, allobarbital, 1--D-arabinofuranosyl cytosine, barbital, bisethylxanthogen, butylated hydroxyanisole, butylated hydroxytoluene, 3,5-di-*tert*-butylcatechol, *tert*-butylhydroquinone, 2-*n*-butylthiophene, cisplatin, clonazepam, cyclophosphamide, dexamethasone, diethyldithiocarbamate, diethyl maleate, diethylnitrosamine, 5,6-dihydro-*2H*-pyran-2-one, dimethyl fumarate, dimethyl maleate, dimethyl itaconate, disulfiram, 1,2-dithiole-3-thione, erucin, erysolin, ethanol, ethoxyquin, 5-ethyl-5-phenylhydantoin, 2-*n*-heptylfuran, -hexachlorocyclohexane, -hexachlorocyclohexane, hexachlorobenzene, 3,4,5,3',4',5'-hexachlorobiphenyl, 2,4,5,2',4',5'-hexachlorobiphenyl, 2,3,5,2',3',5'-hexachlorobiphenyl, interferon-/, i proplatin, isosafrole, lead acetate, *p*-methoxyphenol, methyl acrylate, 3-methylcholanthrene, 2-methylene-4-butyrolactone, methyl selenocyanate, musk xylene, -naphthoflavone, oltipraz, phenobarbital, polychlorinated biphenyl, propylthiouracil, rifampicin, *trans*-stilbene oxide, steptozotocin, sudan, 1,4-bis-[2-(3,5-dichloropyridyloxy)]benzene, tetrachlorodibenzo-*p*-dioxine, 2,3,5,6-tetrafluorophenol, 1-(2-thiazolylazo)-2-naphthol, vinylidene chloride, adriamycin, arsenic, bleomycin, 2-[3-(chloroethyl)-3-nitrosoureido]-D-dioxyglucopyranose, ethacrynic acid, etoposide, hepsulfam, mitomycin C, mitoxantrone, novantrone, oxazaphosphorine, TGF-1 and vincristine. To our knowledge, the majority of GST substrates are either xenobiotics or products of oxidative stress in nature. The common feature for these compounds is that they are involved in the metabolising process or the generation of metabolic cascade. And, some endogenous compounds are also inducers of GST.

Modulation of intracellular GSH content and GST activity is carried out in a replicating hepatic cell line DYD, a human hepatocyte clone established from normal liver tissue. The modulating procedure of the cells is described in Example 1 in detail. By endowing them with enhanced specific detoxification functions, hepatocytes transform toxins more specifically, rapidly and efficiently, thus elevating the efficiency of bioreactor for bioartificial liver support system.

Quantity of hepatocytes inoculated in bioreactor is another important factor which influences the efficiency of current bioreactors. Although no one can figure out the exact number of hepatocyte in bioreactor, the general acceptance for the cell mass is 100-300 gram as extracorporeal liver support. The amount of cells in the present invention is 2.5 - 7.5 billion about 25 - 75 gram per single module. In order to accommodate such a huge number of cells, a special bioreactor configuration is constructed. The functional hepatocytes are allowed to grow to confluence on macroporous microcarriers and then to remove cell attached microcarriers into a bioreactor, where a continuous supply of nutrition and oxygen is guaranteed to maintain the steady functioning of the functional hepatocytes.

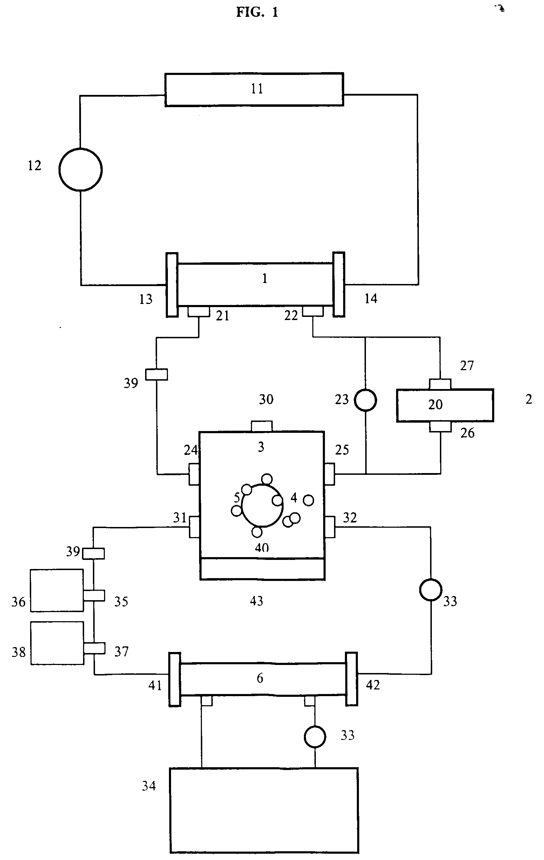

With reference to the embodiment depicted in Fig 1, there is shown a diagrammatical sketch of the system for bioartificial liver support, which is consisted of a plasma separation cartridge for patient circulation **1**, an adsorbent column **2**, a hepatocyte bioreactor **3**, a dialysis cartridge for nutrient / waste circulation and exchange **6**. The hepatocyte bioreactor includes a containment vessel **43**, the said functional hepatocytes **4** that are grown on the macroporous microcarriers **5** at a very high density.

The principle of this invention for liver support is that blood/plasma from a patient flows through plasma separation system **1** intra-capillarily while dialysate circulating through bioreactor **3** extra-capillarily at a definite speed. The dialysate brings the toxins or substances from a patient into bioreactor **3**, where functional hepatocytes **4** are grown on macroporous microcarriers **5**. The functional hepatocytes **4** grown on macroporous microcarriers **5** will bio-transform or metabolise these toxins or substances into less harmful or non-detrimental metabolites. In order to maintain hepatocyte viability and functionality in culture, a nutrient supply and metabolites removal system is connected with hepatocytes bioreactor **3** by the use of a dialysis cartridge **6** that links between bioreactor **3** and medium reservoir **34**. An adsorbent column **2** is connected patient circulation **11** with bioreactor **3**. Activated charcoal is preferable as the adsorbent. Some immunological adsorbents also can be usable.

As for plasma filtration cartridge **1**, a preferred membrane of the cartridge is chosen which allows efficient exchange of a wide spectrum molecular weight compounds, such as nutrients and toxins accumulating in patients with fulminant hepatic failure, to cross between blood/plasma circulation and hepatocyte bioreactor **3**. The chosen membrane of plasma filtration cartridge **1** must be permeable to toxins potentially important for the development of encephalopathy in fulminant hepatic patients, such as ammonia, aromatic amino acids, fatty acids, mercaptans, endogenous benzodiazepines, bile acids, bilirubin, middle molecules, cytokines, endotoxins and the others. Since a lot of toxins known are combined with albumin in the patient blood as a carrier, however, the desired upper molecular weight limit of the membrane would allow the free passage of albumin in order to purify the blood efficiently. Thus, a suitable plasma filtration membrane designed for such a purpose has been installed in this system. Such a membrane is commercially available. The membrane is made of cellulose, cellulose acetate, polyacryronitrile or polysulphone.

The hepatocyte bioreactor **3** includes a containment vessel **43**, wherein the said hepatocytes are grown on macroporous microcarriers **5** in a high density, and a centrifugal pump **40**. The vessel is constructed with plastic material non-toxic to the cells and high mechanical strength. The centrifugal pump **40** is fused at the bottom of the containment vessel **43**. There is an opening **30** on the top of containment vessel **43** as hepatocyte injection, and four openings on the two sides of containment vessel **43** designated as dialysate inlets **24, 31** and dialysate outlets **25, 32**. The opening on the top **30** is used as injection port of macroporous microcarriers into containment vessel **43**. At the mouth of each inlet and outlet, a filter is fixed to avoid cellular entrance into dialysate circulation. Meanwhile, a pressure sensor 39 in dialysate circulation circuit monitors pressure change indicating the circuit clotting.

The composition of the dialysate circulation is culture medium William's E supplemented with 10 - 30% normal human serum or plasma. The purpose of supplementing normal human serum or plasma is to compensate the falling hepatic synthesis function by infusing the human native blood components, to increase the colloid osmosis in the dialysate, and to facilitate the mass exchange between the patient circulation and dialysate circulation.

To the bottom of containment vessel **43** is fixed a centrifugal pump that makes macroporous microcarriers in suspension condition. Macroporous microcarriers serve as a matrix for anchoring of hepatocytes. In the preferred embodiment, containment vessel **43** is made from polypropylen, polycarbonate or polysulfone. Macroporous microcarrier is supplied by Pharmacia LKB Biotechnology as trade names of Cytodex™, Cytopore™ and Cytoline™. The diameter of macroporous microcarrier is preferably 30 - 500 m, and most preferably 100 - 300m. The microcarrier is made of cellulose, cross-linked dextran, polyethylene and/or silica.

The viability and functionality of hepatocytes in bioreactor is maintained by a nutrition supply and metabolite waste removal system. This system includes a dialysis cartridge **6** for mass exchange between hepatocyte bioreactor 3 and medium reservoir **34**, a pH sensor **35** and a dissolved oxygen sensor **37**, a syringe to inject pH adjusting solution **36** and oxygenator system **38** in order to adjust pH value and dissolved oxygen concentration in intra-capillary circulating fluid. Dialysate flows through the extracapillary of dialysis cartridge **6**. Dialysate in this part is cell culture medium William's E without any plasma protein supplements.

Hepatocyte culture on macroporous microcarrier makes it possible the practical high yield culture of anchorage-dependent cells. In macroporous microcarriers culture hepatocytes grow not only as monolayer on the surface of microcarriers but also on the inner pores of the microcarriers. By using microcarriers in suspension or perfusion culture system it is possible to achieve yields of ∼ 10⁷ up to 10⁸ cells/ ml.

Hepatocytes bioreactor is the central part of a bioartifical liver support system. Hepatocytes are enclosed in containment vessel **43**, and suspended by a centrifugal pump **40**. The material of containment vessel **43** uses preferentially polypropylen, polycarbonate, polystyrene or polysulfone. The advantages for such kind of material in vessel construction are:
1. It is proved to be non-toxic to cell viability and metabolism.
2. It can be easily sterilised.
3. Its mechanical strength can match the vibration when centrifugal pump **40** works.

At the two sides of containment vessel **43** are dialysate inlets **24, 26** and outlets **25, 27**. They are specially manufactured to match the standard plastic fittings for routine dialysis products. Besides dialysate inlets and outlets, a filter (not shown in the figure) is mounted at the each mouth of inlets and outlets so that it prevents cells and their pieces or even larger particles from entering from blood circulation. The pore size of the filter is below 10 µm, preferred 1 - 5µm, for successful preventing leakage of cellular component into and minimal interfering with dialysate circulation. The filter is changeable in the case of inlets or outlets clotting up by particles. Materials for the filter are made from Millipore cellulose membranes.

Tubes connecting different components of the whole system are silicone tubing, which have a similar standard as routinely used in dialysis treatment with the features being manufactured in same manner.

Macroporous microcarriers provide both an external surface and an interior space which can be anchored by hepatocytes grown in suspension. In this way, macroporous microcarriers enhance productivity by increasing cell density per reactor volume. The well grown hepatocytes on macroporous microcarriers can reach as high as ∼10⁸/ml.

Macroporous microcarrier culture starts with inoculation of the said hepatocytes **4** onto microcarriers **5**. Hepatocytes **4** in a logarithmic growth phase and in a good nutritional state are harvested by protease digestion and transferred to the flasks to allow cellular attachment to the microcarriers **5** with a even distribution. After 5 hours, add culture medium to the final volume. In order to obtain sufficient cell number, a fluidized bed culture system is used to scale-up the productivity. The high cell density afforded by macroporous microcarriers **5** results in rapid consumption of any available metabolites. Therefore, a steady state should always be maintained at the set level by a medium perfusion and oxygen supply system. Cell growth can be monitored by biochemical determinations and cell counting. Harvest cells **4** when it becomes confluence on macroporous microcarriers **5**.

Macroporous microcarriers **5** with well-grown hepatocytes **4** are transferred to containment vessel **43** of bioreactor **3** in sterile from top opening **30**. The amount of transferred cells may be at ranged from 5 to 7.5 x 10¹⁰, usually 5 x 10¹⁰. The cell amount in the bioreactor is largely dependent on the clinical requirement according to the severity of patient condition.

After transferring macroporous microcarriers, bioreactor **3** is initially filled with fresh medium containing 10-30% human serum or plasma William's E medium. The medium is needed also to fill the circuit connected with bioreactor **3** as dialysate flows through the extracapillary space of plasma filtration system **1**. The dialysate is circulated by a peristaltic pump **23**.

Functional hepatocytes are maintained by a continuous flow of nutrient medium which crosses dialysis membrane **6**, and toxic cell waste products diffuse across dialysis membrane **6** to medium reservoir **34**. The preferred membrane exclusively allow the free passage of small molecules such as amino acids and mercapturic acids between intracapillary and extracapillary spaces. But dialysis membrane **6** is not permeable to high molecular weight compound such as plasma proteins. The suitable membrane for a purpose has a molecular cut-off ranging from 12,000 - 14,000 kd. Cellulose membrane is the best candidate for such a membrane. Other membranes that can be used with the present invention include polysulphon, polypropylene, and etc. The composition of dialysate is William's E without serum. Medium reservoir is changed in a definite time while the patient circulation is started.

Nutrient medium from medium reservoir **34** crosses the semi-permeable membrane of dialysis cartridge **6** to feed the hepatocytes **4** on microcarriers **5**. Cell waste products and toxin conjugates are removed by dialysate, and then the dialysate is changed periodically. In this way, the system replaces the excretory function of the liver.

### Example 1:

### Establishment of a functional human hepatocyte cell line and its modulation of GSH content and GST activity

Cisplatin ( *cis*-diaminedichloroplatinum; *cis*-DDP) is a chemo-therapeuitc drug to treat a wide spectrum of tumors. The mechanism of this drug is to kill the cells in a way of causing cytotoxicity. The established human liver cell line DYD is used to modulate its GSH content and GST activity in order to obtain specially functional hepatocytes characterised by higher capacity of detoxification functions than its parental cell.

All cell culture reagents, including William's E medium, foetal calf serum (FCS), penicillin-streptomycin, sodium pyruvate and L-glutamine, were purchased from Gibco Life Technologies Ltd. All other chemicals were products of Sigma (St, Louis, USA) unless mentioned. The established human liver DYD cells were grown as a mono-layer culture in William's E supplemented with 10% (v/v) FCS in an atmosphere of 5% CO₂ in air. Cells were subcultured once a week at a split ratio of 1:3 using trypsin and EDTA. Cisplatin was purchased from Sigma, St.Lois, USA. It was dissolved in Hans' solution. Stock solution was kept in -20 °C. The stock solution was diluted according to the concentrations needed with culture medium.

DYD cells were treated with 0.5 µg/ml cisplatin for 24 hours in the medium supplemented with serum weekly. During this period, the medium without cisplatin was used as routinely medium to culture the cells. This procedure was repeated by the increasing concentrations of cisplatin from 0.5 µg/ml to 5.0 µg/ml during ten months treatment. The cells were cloned and stored the cloned cells in liquid nitrogen. A set of clones were designated with initial DYD-, following with the time of drug exposure in weeks.

For GSH and GST determination, DYD cells and their clones were grown in 24-well plates [Costar, USA] Glutathione and glutathione S-transferase were determined in every batch of cloned cells. Total intracellular glutathione determination was measured fluorimetrically. GST activity in cell homogenates by direct spectrophotometry using 50 µM 1-chloro-2,4-dinitrobenezene (CDNB).

### Results:

Table 1 gives the GSH contents and GST activities in three different clones of DYD cell. GSH contents in these three clones were compared with that of parental cells as shown in Table 1. GSH levels were elevated in all three clones and well related to the duration of drug exposure. GST activities were enhanced at different levels in these clones. In combination with GSH, the increased GST activities might perform an important part in conjugation of toxins to GSH.

**Tab.1**

| GSH contents and GST activities in parental and modulated cells | | | | |
|---|---|---|---|---|
| | Control | DYD- 10 | DYD-30 | DYD-50 |
| Toxin exposure time | 0 | 10 weeks | 30 weeks | 50 weeks |
| GSH Content GSH Content (nmol/mg protein) | 20 - 40 | 100 - 150 | 300 - 400 | 300 - 500 |
| GST Activity (nmol/mg pro./min) | 5 - 15 | 15 - 20 | 20 - 30 | 20 - 35 |

### Example 2:

### Characterisation of the established functional cell line and its comparison with human primary culture of hepatocytes and immortalised cells

The present invention introduced functional hepatocyte clones are established from human liver regenerating tissue, but possess an enhanced GSH content and GST activity. Table 2 indicates the GSH content and GST activity in this cell line, an immortalised human liver cell line (taking Hep G2 cell as an example) and primary culture of hepatocytes.

Primary culture of hepatocytes are freshly isolated from liver tissue. Hepatocytes were digested with 0.25% collegenase and cells then dispersed into balanced salt solution. After washed and filtered with a cell mesh, the cells were seeded in Petri dishes at a concentration of 5 x 10⁶ in 10 cm dish. DMEM medium is used to culture the cells with 10% FCS. Hep G2 cells and DYD-50 cells culture are the same to Example 1.

**Table 2.**

| GSH contents and GST activities in established and primary hepatocytes | | | |
|---|---|---|---|
| | Hep G2 cells * | Primary Culture ** | DYD-50 |
| GSH Content (nmol/mg protein) | 10 - 40 (24 hours subculture) | 20 - 25 (24 hours culture) | 100 - 500 (24 hours subculture) |
| GST Activity (nmol/mg pro./min) | 5 - 15 (24 hours subculture) | 15 - 20 (24 hours culture) | 20 - 35 (24 hours subculture) |

| | | | |
|---|---|---|---|
| * Grant M.H, et al: Biochemical Pharmacology, 37(17): 3365-68 1988 Doostrar H, et al: Xenobiotica, 20(4):435-41 1990 | | | |
| ** Grant M.H, et al: Biochemical Pharmacology, 36(14): 2311-16 1987 | | | |

### Example 3:

### Scale-up of established functional cells on macroporous microcarriers

Macroporous microcarriers are purchased from Pharmacia, at the trade name of Cytopore™ and Cytoline™. Cytopore™ is consisted of cellulose and Cytoline™ of polyethylene and silica. Before use of both microcarriers, it is necessary to hydrate and swell the dry microcarriers in a fresh distilled water or PBS. Replace the distilled water with culture medium to equilibrate the microcarriers overnight under culture conditions. In order to provide a homogenous culture environment and to encounter less shearing forces, a perfusion culture system is used to support high density cell culture. In this experiment, fluidized or packed bed reactor is optimal reactor for Cytoline microcarriers. This type of reactor combines the high density culture of the perfusion technique with low shear forces.

Cell suspension was prepared by trypsin digestion of cells grown on flasks in exponential growth phase and in a good nutritional state. Added microcarriers at a ratio 2 x 10⁶/ml microcarrier packed volume in large flasks. Put the flasks into the incubator during the attachment phase, and shaken gently every 30 minutes to obtain uniform cell distribution on the microcarriers. After 5 hours added sufficient culture medium and put microcarriers in a bioreactor. In a fluidized bed culture, the maximum volume of packed microcarriers that could be used was 50% of reactor volume. In a packed bed culture, the maximum volume of packed microcarriers that could be used was 75% of the reactor volume.

In order to maintain high cell density in the bioreactor, toxic metabolites must not be allowed to accumulate and pH values should be maintained at the set level. The desired level of oxygen concentration should also be maintained. Perfused medium was changed periodically.

Usually, it takes 1-2 weeks for DYD hepatocytes to become confluent under the perfusion culture conditions. Upon the harvest of cells, the density could reach 10⁷ - 10⁸/ml in a fluidized bed bioreactor.

### 4. Brief Explanation of the Drawings

Fig. 1 is a general structure of the specific Extracorporeal Liver Assist device (sELAD) that uses special functional hepatocytes with enhanced detoxification functions enclosed this invention.

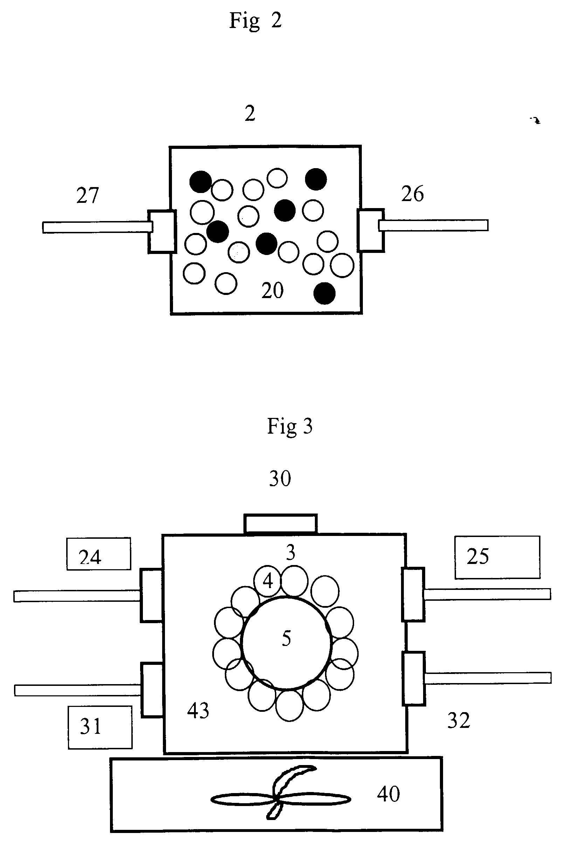

Fig. 2 is a representation of adsorbents column

Fig. 3 is a representation of hepatocyte bioreactor
1. Plasmafiltration cartridge for patient circulation
2. Adsorbent perfusion tank
3. Hepatocyte attached microcarrier bioreactor
4. Hepatocytes
5. Microcarriers
6. Dialysis cartridge for nutrient/waste circulation
11. Patient subject
12. Pump
13. Blood inlet
14. Blood outlet
20. Adsorbents
21. Dialysate-outlet
22. Dialysate-inlet
23. Pump
24. Dialysate-inlet
25. Dialysate-outlet
26. Dialysate-inlet
27. Dialysate-outlet
30. Opening of bioreactor
31. Bioreactor circulation inlet
32. Bioreactor circulation outlet
33. Pump
34. Medium reservoir
35. pH sensor
36. pH adjusting solution syringe
37. Dissolved oxygen sensor
38. Oxygenator
39. Pressure sensor
40. Centrifugal pump
41. Dialysis circulation outlet
42. Dialysis circulation inlet
43. Containment vessel

The features disclosed in the foregoing description, in the claims and/or in the accompanying drawings may, both separately and in any combination thereof, be material for realising the invention in diverse forms thereof.

## Claims

1. A set of functional human liver cell clones (DYD-) derived from normal hepatocyte cell line (DYD) exhibits enhanced detoxification activities as characterised by increased GSH content and elevated GST activity.

2. The set of functional human liver cell clones of claim 1, wherein said increased GSH content and elevated GST activity are compared to immortalised human hepatocytes cell lines and primary human hepatocytes culture isolated from liver tissue.

3. The cell line of claim 1, wherein said functional hepatocytes have GSH content is above 50 nmol/mg protein or 50 nmol/10⁶ cells after 24 hours passage.

4. The cell line of claim 1, wherein said functional hepatocytes have GST activity is above 15 nmol/mg protein/min or 80 nmol/10⁶ cells /min with CDNB as substrate after 24 hours passage.

5. A method to obtain the set of functional human liver cell clones of claim 1, which comprises a process of exposing normal hepatocyte cell line to toxin-challenging culture medium condition and modulating expressions of related enzymes with inducers in the said cell line.

6. The method claimed in claim 5, wherein the inducers possess either carcinogenic or cytotoxic nature.

7. An extracorporeal liver assist device, which has a bioreactor containing functional human liver cell clones of claim 1.

8. The extracorporeal liver assist device in claim 7, wherein said functional human liver cell clones are grown preferentially on macroporous microcarriers or other biocompatible support matrix system, for example, capillaries or ceramics.

9. The extracorporeal liver assist device in Claim 7, wherein the device comprises plasma filtration circulation system and dialysis cartridge circulation system.

10. The extracorporeal liver assist device in claim 9, wherein the plasma filtration circulation system has an adsorbent column.
